# EUROPÄISCHE PATENTSCHRIFT

(11) **EP 2 482 786 B1**
(45) Veröffentlichungstag und Bekanntmachung des Hinweises auf die Patenterteilung: **04.05.2016**
(21) Anmeldenummer: 11784579.2
(22) Anmeldetag: 28.05.2011
(51) Int. Cl.: B65D 1/09, A61M 31/00

(54) **EINMAL-APPLIKATOR**
DISPOSABLE APPLICATOR
APPLICATEUR UNIQUE

(30) Priorität: 31.07.2010 DE 102010033015
(43) Veröffentlichungstag der Anmeldung: 08.08.2012
(73) Patentinhaber: Gaplast GmbH, 82442 Altenau (DE)
(72) Erfinder: KNEER, Roland, 82490 Farchant (DE)
(74) Vertreter: Flosdorff, Jürgen
(86) Internationale Anmeldenummer: PCT/DE2011/001128
(87) Internationale Veröffentlichungsnummer: WO 2012/016553

(56) Entgegenhaltungen:
- EP-A1- 1 526 092
- DE-A1- 1 441 377
- US-A- 2 552 100
- US-A- 2 744 528
- US-A- 3 114 369
- US-A- 4 072 249
- US-A- 6 112 752

## Beschreibung

Die Erfindung betrifft einen Einmal-Applikator zur Abgabe einer Füllsubstanz, mit einem Aufnahmeraum für die Substanz, der mit einem Auslaß versehen ist.

Der Einmal-Applikator ist insbesondere dazu vorgesehen, die gesamte Füllsubstanz in einem einzigen Vorgang abzugeben. Es liegt aber auch im Rahmen der Erfindung, dass der Behälterinhalt in zwei oder mehr Teildosierungen ausgebracht werden kann. Bei der Füllsubstanz handelt es sich bevorzugt um einen zähflüssigen oder cremigen Behälterinhalt, wobei der Einmal-Applikator aber auch zur Abgabe einer dünnflüssigen Substanz geeignet ist.

Der Behälterinhalt kann beispielsweise eine pharmazeutische Substanz sein, die in die Nase eines Benutzers eingebracht wird, ohne dass die Erfindung hierauf beschränkt ist.

Derartige Einmal-Applikatoren, die ein kleines Volumen in der Größenordnung von einem Kubikzentimeter oder weniger haben können, haben im allgemeinen die Form eines kleinen Fläschchens und werden zur Abgabe des Behälterinhalts seitlich zusammen gedrückt, um den Behälterinhalt auszupressen. Dabei bleibt bisher eine nicht unerhebliche Restmenge in dem zusammengedrückten Behälter zurück, die nicht ausbringbar ist.

US 3,114,369 A offenbart eine Wegwerfampulle, die eine nach außen gewölbte Wand und eine gegenüber liegend Wand hat. Die gewölbte Wand hat an den seitlichen Endbereichen zwei schräg nach innen verlaufende, geradlinige Wandabschnitte, mit denen die gewölbte Wand an der gegenüber liegenden Wand angesetzt ist. Diese schrägen Wandabschnitte sind nicht umlaufend, sondern erstrecken sich lediglich an den gegenüber liegenden Längsseiten der Ampulle, und sie greifen nicht in den Hohlraum der gewölbten Wand ein.

US 2,552,100 A offenbart eine Ampulle, deren Aufnahmeraum ebenfalls eine nach außen gewölbte Wand und eine gegenüber liegende Wand aufweist. Die gegenüber liegende Wand hat an ihrem einem Auslassröhrchen gegenüber liegenden rückwärtigen Abschnitt eine konvexe Wölbung, die nicht umlaufend ausgebildet ist.

US 4,072,249 A offenbart einen Behälter für kleine Mengen einer Substanz, der zwei gewölbte Wände hat, von denen eine in die andere hinein gedrückt wird. Dabei ist kein umlaufender Steg vorgesehen, der in den Randbereich einer der gewölbten Wände eingreift.

US 2 744 528 A offenbart eine Spritzenampulle, die eine gewölbte, zusammen drückbare Wand enthält, um die Substanz aus dem Aufnahmeraum durch eine Nadel abzugeben. Der Spritzenkörper, der die gegenüber liegende Wand des Aufnahmeraums bildet, hat zwei kurze konische Stege, während die übrigen Umfangsbereiche eben sind. Wenn der Behälterinhalt ausgebracht wird baucht die gewölbte Wand nach beiden Seiten seitlich aus, so dass sich in diesen überstehenden Bereichen zwangsläufig Behälterinhalt ansammelt, der nicht ausgebracht werden kann.

DE 14 41 377 A1 offenbart eine Spritzampulle, die einen Aufnahmeraum für eine abzugebende Substanz mit einer Injektionsnadel enthält, die sich im Ausgangszustand der Spritzampulle innerhalb des Aufnahmeraums befindet. Der Aufnahmeraum ist durch eine im wesentlichen ebene Wand und durch eine gegenüber liegende Wand begrenzt, die als Faltenbalg ausgebildet ist. Der Faltenbalg besteht aus mehreren Stufen, die sich bei der Abgabe des Behälterinhalts zusammen falten lassen. Dabei legt sich ein Abschnitt des Faltenbalgs dicht an die Innenwand des breitesten Abschnitts und an einen spitzen konischen Steg an, der um den gesamten Hohlraum umlaufend an der ebenen Wand angeformt ist. Der Faltenbalg hat in seinem mittleren Bereich eine größere Wandstärke als da, wo er zusammen gefaltet wird, da auf den mittleren Bereich zur Abgabe des Behälterinhalts ein Druck ausgeübt wird, durch den die Injektionsnadel über ihre kreisförmige Basis aus dem Aufnahmeraum heraus gedrückt wird.

Der vorliegenden Erfindung liegt die Aufgabe zugrunde, einen Einmal-Applikator anzugeben, bei dem praktisch der gesamte Behälterinhalt - bis auf eine zu vernachlässigbare Restmenge - ausbringbar ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass der Einmal-Applikator einen Aufnahmeraum für die Füllsubstanz aufweist, der aus einer nach außen gewölbten Wand und einer gegenüber liegenden Wand besteht, wobei die letztere mit einem umlaufenden Steg versehen ist, der in den Hohlraum der gewölbten Wand an dessen Rand eingreift, und dass die gewölbte Wand so geformt und bemessen ist, dass sie an den Steg und den dazwischen liegenden Bereich der gegenüber liegenden Wand glatt anpressbar ist. Dabei liegt im zusammen gepressten Zustand die gewölbte Wand im wesentlichen ohne verbleibenden Zwischenraum an dem Steg und der gegenüber liegenden Wand an, so dass praktisch der gesamte Inhalt aus dem Aufnahmeraum heraus gedrückt wird, ohne dass eine nennenswerte Restmenge zurück bleiben kann, da sich die gewölbte Wand glatt, ohne Faltenbildung, um den Steg herum an die gegenüber liegende Wand anlegt. Der Steg, der vorzugsweise querschnittlich eine im wesentlichen konische Form hat, mit gerundeter Spitze und leicht gerundeten Flanken, füllt dabei den Raum aus, der sich beim Zusammendrücken einer gewölbten Wand an deren äußeren Rand zwangsläufig ausbildet. Da dieser Raum durch den Steg ausgefüllt ist, kann dort keine Restmenge des Behälterinhalts zurück bleiben.

Die gewölbte Wand hat vorzugsweise die Form einer Kugelkapp®, wobei der Steg dann kreisförmig von der gegenüber liegenden Wand absteht.

Die gewölbte Wand kann aber beispielsweise auch einen ovalen Grundriss haben, wobei der umlaufende Steg dann im Grundriss ebenfalls eine entsprechende ovale Form hat.

Die gegenüber liegende Wand, die den Aufnahmeraum begrenzt, ist vorzugsweise eben. Es liegt aber auch im Rahmen der Erfindung, dass diese Wand ebenfalls vorzugsweise nach außen gewölbt sein kann, wobei diese Wölbung dann flacher verläuft, so dass sich die andere Wand glatt anpressen lässt.

Weiter sieht die Erfindung vor, dass die nach außen gewölbte Wand und die gegenüber liegenden Wand jeweils einen ebenen umlaufenden Rand aufweisen, und dass diese Ränder aneinander befestigt sind.

Vorzugsweise ist dabei vorgesehen, dass diese Randbereiche miteinander verschweißt sind, vorzugsweise durch Ultraschallschweißen, und dass sie an ihren einander zugewandten Flächen im Ausgangszustand - vor dem Verschweißen - mit kleinen Rillen versehen sind.

Der Auslass des Einmal-Applikators, durch den die Füllsubstanz abgegeben wird, wird nach einem weiteren Vorschlag der Erfindung durch ein Röhrchen gebildet, das an der gewölbten Wand angesetzt und zu dem Aufnahmeraum hin offen ist.

Das freie Ende des Röhrchens ist vorzugsweise durch einen abreißbaren Stopfen oder Pin verschlossen, der mit einer dünnen Abreißnaht innen an dem Röhrchen angesetzt ist. Zur Abgabe des Behälterinhalts wird der Pin z.B. durch Drehen um seine Längsachse von dem Röhrchen entfernt. Da die Abreißnaht sich im Inneren des Röhrchens befindet, steht beim Gebrauch kein verbleibender Grat von dem Röhrchen vor, so dass das Röhrchen keine Verletzung hervorrufen kann, wenn es beispielsweise in die Nase eines Benutzers eingeführt wird.

Mit großem Vorteil wird außerdem vorgeschlagen, dass der Einmal-Applikator einstückig im Spritzgussverfahren hergestellt wird, wobei die Ränder der beiden Wände des Aufnahmeraums mit einem Filmscharnier verbunden sind. Dabei wird zunächst der Hohlraum der gewölbten Wand mit Behälterinhalt gefüllt, woraufhin die gegenüber liegende Wand an dem Filmscharnier umgeklappt und mit ihrem Rand auf den Rand der gewölbten Wand aufgelegt wird, wobei der umlaufende Steg für die Zentrierung der beiden Gehäusehälften sorgt. Anschließend werden die Ränder verschweißt. Insbesondere dann, wenn die gegenüber liegende Wand eben ist, kann damit der gesamte Aufnahmeraum vollständig befüllt werden.

Als Material für den Einmal-Applikator sind beispielsweise Polyolefine wie PP oder PE geeignet, ohne dass die Erfindung hierauf beschränkt ist.

Die gewölbte Wand hat erfindungsgemäß eine geringere Wandstärke als die gegenüber liegende Wand und die Ränder, damit der Aufnahmeraum problemlos zusammen gedrückt werden kann. Beispielsweise beträgt die Wandstärke der gewölbten Wand 0,2 bis 0.3 mm, während die gegenüber liegende, vorzugsweise ebene Wand und die beiden Ränder 0,6 mm dick sein können, womit sie die zum Verschweißen erforderliche Stabilität haben.

Bei dem erfindungsgemäßen Einmal-Applikator ist praktisch der gesamte Behälterinhalt auspressbar, was insbesondere bei zähflüssiger oder cremiger Füllsubstanz wünschenswert ist, die nicht durch Schwerkraft aus dem Applikator auslaufen kann. Ein weiterer großer Vorteil des erfindungsgemäßen Applikators liegt darin, dass er mit geringen Kosten einstückig im Spritzgussverfahren herstellbar ist.

Weitere Einzelheiten der Erfindung ergeben sich aus der nachfolgenden Beschreibung sowie anhand der Zeichnungen. Dabei zeigen:
- Figur 1: eine perspektivische Ansicht des fertigen Einmal-Applikators im vergrößerten Maßstab;
- Figur 2: eine Aufsicht auf den Rohling des Einmal-Applikators im aufgeklappten Ausgangszustand;
- Figur 3: einen Schnitt A-A in Figur 2;
- Figur 4: einen Schnitt B-B in Figur 2;
- Figur 5: eine Aufsicht auf den zusammen gesetzten Einmal-Applikator;
- Figur 6: einen Schnitt D-D in Figur 5;

- Figur 7: eine Einzelheit C in Figur 4 und
- Figur 8: einen Längsschnitt durch den Einmal-Applikator im ausgedrückten Zustand.

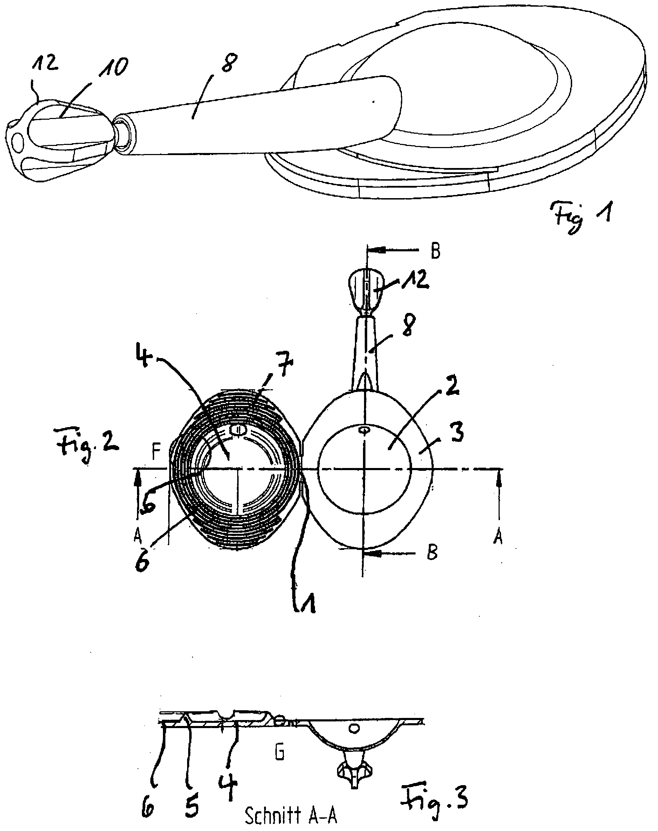

Figur 2 zeigt den Einmal-Applikator in dem Zustand, in dem er die Spritzgussform verlässt. Der Einmal-Applikator ist aus zwei Behälterhälften zusammen gesetzt, die durch ein Filmscharnier 1 miteinander verbunden sind. Die in Figur 2 rechte Behälterhälfte enthält eine gewölbte Wand 2, die die Form einer Kugelkappe hat, die von einem ebenen Rand 3 umgeben ist.

Die andere Behälterhälfte besteht aus einer ebenen Wand 4, von der ein kreisringförmiger, im Querschnitt etwa konischer Steg 5 vorsteht, woran sich außen ebenfalls ein ebener Rand 6 anschließt. Die Außenkonturen der Ränder 3 und 6 sind identisch. Die Ränder 3 und 6 sind mit Rillen 7 versehen.

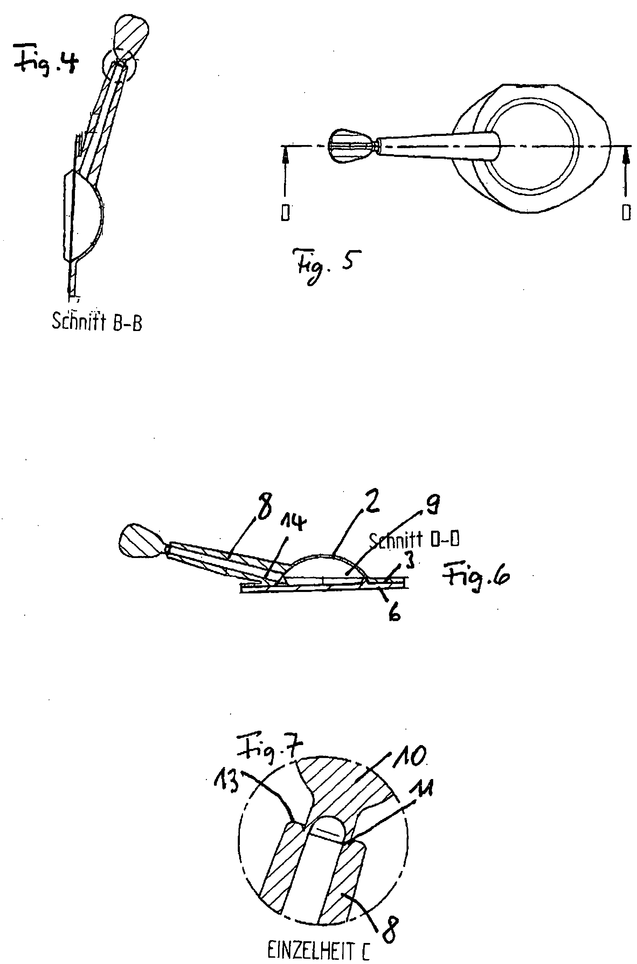

An der gewölbten Wand 2 ist ein Röhrchen 8 angeformt, das zu dem Aufnahmeraum 9 des Einmal-Applikators hin offen ist. Das Röhrchen 8 ist vor Abgabe des Behälterinhalts durch einen Abreißpin 10 verschlossen, der mit einer dünnen Abreißnaht 11 im Inneren des Röhrchens 8 angesetzt ist (Figur 7). Der Abreißpin 10 hat einen profilierten Kopf 12, mit dem der Abreißpin 10 leicht von dem Röhrchen 8 abdrehbar ist. Dabei ist die Abreißnaht gegenüber der Stirnfläche 13 des Röhrchens 8 zurück versetzt, so dass kein Grat über die Stirnfläche 1 3 hinaus vorsteht.

Der kreisringförmige Steg 5 hat im Bereich des Kanals 14 des Röhrchens 8 eine kleine Aussparung, so dass der Steg 5 nicht den Austritt des Behälterinhalts durch das Röhrchen 8 behindert.

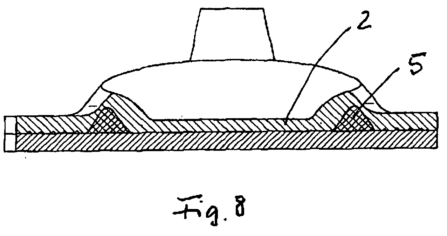

Figur 8 zeigt den ausgedrückten Zustand des Einmal-Applikators. Wenn eine gewölbte Wand entgegen ihrer Wölbung nach innen gedrückt wird, bildet sich an ihrem äußeren Rand zwangsläufig ein ringförmiger Hohlraum, der von einem Finger des Benutzers nicht gänzlich zusammenpressbar wäre. Ohne den kreisringförmigen Steg 5 würde hier zwangsläufig Behälterinhalt zurück bleiben, der nicht ausbringbar wäre. Bei dem erfindungsgemäßen Einmal-Applikator wird dieser Randbereich von dem Steg 5, der im wesentlichen eine konische Querschnittsform hat, ausgefüllt, so dass praktisch der gesamte Behälterinhalt ausbringbar ist.

## Patentansprüche

1. Einmal-Applikator zur Abgabe einer Füllsubstanz mit einem Aufnahmeraum für die Substanz, der mit einem Auslass versehen ist, wobei der Aufnahmeraum (9) eine nach außen gewölbte Wand (2) und eine gegenüber liegende Wand (4) aufweist, wobei die gegenüber liegende Wand (4) mit einem umlaufenden Steg (5) versehen ist, der in den Hohlraum der gewölbten Wand (2) an dessen Rand eingreift,
**dadurch gekennzeichnet,**
**dass** der Steg (5) querschnittlich eine im wesentlichen konische Form hat mit gerundeter Spitze und leicht runden Flanken,
**dass** die gewölbte Wand (2) so geformt ist, dass sie an den Steg (5) und den dazwischen liegenden Bereich der gegenüber liegenden Wand (4) glatt anpressbar ist, und
**dass** die gewölbte Wand (2) eine geringere Wandstärke hat als die gegenüber liegende Wand (4) und deren Ränder (3, 6).

2. Einmal-Applikator nach Anspruch 1,
**dadurch gekennzeichnet,**
**dass** die gewölbte Wand (2) die Form einer Kugelkappe hat.

3. Einmal-Applikator nach Anspruch 1,
**dadurch gekennzeichnet,**
**dass** die gewölbte Wand einen ovalen Grundriss hat.

4. Einmal-Applikator nach einem der Ansprüche 1 bis 3,
**dadurch gekennzeichnet,**
**dass** die gegenüber liegende Wand (4) eben ist.

5. Einmal-Applikator nach einem der Ansprüche 1 bis 3,
**dadurch gekennzeichnet,**
**dass** die gegenüber liegende Wand ebenfalls gewölbt ist.

6. Einmal-Applikator nach einem der Ansprüche 1 bis 5,
**dadurch gekennzeichnet,**
**dass** die nach außen gewölbte Wand (2) und die gegenüber liegende Wand (4) jeweils einen ebenen umlaufenden Rand (3,6) aufweisen und dass diese Ränder (3,6) aneinander befestigt sind.

7. Einmal-Applikator nach Anspruch 6,
**dadurch gekennzeichnet,**
**dass** die Ränder (3,6) miteinander verschweißt sind.

8. Einmai-Applikator nach einem der Ansprüche 1 bis 7,
**dadurch gekennzeichnet,**
**dass** der Auslass durch ein Röhrchen (8) gebildet ist, das an der gewölbten Wand (2) angeformt und zu dem Aufnahmeraum (9) hin offen ist.

9. Einmal-Applikator nach Anspruch 8,
**dadurch gekennzeichnet,**
**dass** das freie Ende des Röhrchen (8) durch einen abreißbaren Pin (10) verschlossen ist, der innen an dem Röhrchen (8) angesetzt ist.

10. Einmal-Applikator nach einem der Ansprüche 1 bis 9,
**dadurch gekennzeichnet,**
**dass** der Einmal-Applikator einstückig im Spritzgussverfahren hergestellt ist, wobei die Ränder (3,6) der beiden Wände (2,4) mit einem Filmscharnier (1) verbunden sind.

## Claims

1. A disposable applicator for dispensing a filler substance with a receiving space for the substance, which is provided with an outlet, wherein the receiving space (9) includes an outwardly domed wall (2) and an opposing wall (4), wherein the opposing wall (4) is provided with a peripheral web (5), which engages in the cavity of the domed wall (2) at its edge, **characterised in that** the web (5) has a substantially conical shape in cross-section with a rounded tip and gently rounded flanks, that the domed wall (2) is so shaped that it may be smoothly pressed against the web (5) and the region of the opposing wall (4) situated therebetween and that the domed wall (2) has a smaller wall thickness than the opposing wall (4) and its edges (3, 6).

2. A disposable applicator as claimed in Claim 1, **characterised in that** the domed wall (2) has the shape of a spherical cap.

3. A disposable applicator as claimed in Claim 1, **characterised in that** the domed wall has an oval outline.

4. A disposable applicator as claimed in one of Claims 1 to 3, **characterised in that** the opposing wall (4) is flat.

5. A disposable applicator as claimed in one of Claims 1 to 3, **characterised in that** the opposing wall is also domed.

6. A disposable applicator as claimed in one of Claims 1 to 5, **characterised in that** the outwardly domed wall (2) and the opposing wall (4) have a respective flat, peripheral edge (3, 6) and that these edges (3, 6) are fastened to one another.

7. A disposable applicator as claimed in Claim 6, **characterised in that** the edges (3, 6) are welded together.

8. A disposable applicator as claimed in one of Claims 1 to 7, **characterised in that** the outlet is constituted by a small tube (8), which is integrally formed on the domed wall (2) and is open towards the receiving space (9).

9. A disposable applicator as claimed in Claim 8, **characterised in that** the free end of the small tube (8) is closed by a tearable pin (10), which is attached to the interior of the small tube (8).

10. A disposable applicator as claimed in one of Claims 1 to 9, **characterised in that** the disposable applicator is produced in one piece in an injection moulding method, wherein the edges (3, 6) of the two walls (2, 4) are connected together by an integral hinge (1).

## Revendications

1. Applicateur à usage unique servant à distribuer une substance de remplissage comprenant un compartiment de réception pour la substance, qui est pourvu d'une sortie, sachant que le compartiment de réception (9) présente une paroi (2) bombée vers l'extérieur et une paroi (4) opposée, sachant que la paroi (4) opposée est pourvue d'une entretoise (5) périphérique, qui vient en prise avec la cavité de la paroi (2) bombée au niveau du bord de la cavité,
**caractérisé en ce**
**que** l'entretoise (5) présente, dans la section transversale, une forme essentiellement conique, comprenant une pointe arrondie et des flancs légèrement ronds,
en ce que la paroi (2) bombée est formée de telle sorte qu'elle peut être comprimée de manière lisse contre l'entretoise (5) et la zone intercalée de la paroi (4) opposée, et
en ce que la paroi (2) bombée présente une épaisseur de paroi inférieure à la paroi (4) opposée et à ses bords (3, 6).

2. Applicateur à usage unique selon la revendication 1,
**caractérisé en ce**
**que** la paroi (2) bombée présente la forme d'une trappe sphérique.

3. Applicateur à usage unique selon la revendication 1,
**caractérisé en ce**
**que** la paroi bombée présente un contour de base ovale.

4. Applicateur à usage unique selon l'une quelconque des revendications 1 à 3,
**caractérisé en ce**
**que** la paroi (4) opposée est plane.

5. Applicateur à usage unique selon l'une quelconque des revendications 1 à 3,
**caractérisé en ce**
**que** la paroi opposée est également bombée.

6. Applicateur à usage unique selon l'une quelconque des revendications 1 à 5,
**caractérisé en ce**
**que** la paroi (2) bombée vers l'extérieur et la paroi (4) opposée présentent respectivement un bord (3, 6) périphérique plan, et en ce que lesdits bords (3, 6) sont fixés les uns contre les autres.

7. Applicateur à usage unique selon la revendication 6,
**caractérisé en ce**
**que** les bords (3, 6) sont soudés les uns aux autres.

8. Applicateur à usage unique selon l'une quelconque des revendications 1 à 7,
**caractérisé en ce**
**que** la sortie est formée par un petit tube (8), qui est formé au niveau de la paroi (2) bombée et qui est ouvert en direction du compartiment de réception (9).

9. Applicateur à usage unique selon la revendication 8,
**caractérisé en ce**
**que** l'extrémité libre du petit tube (8) est fermée par une broche (10) arrachable, qui est posée côté intérieur au niveau du petit tube (8).

10. Applicateur à usage unique selon l'une quelconque des revendications 1 à 9,
**caractérisé en ce**
**que** l'applicateur à usage unique est fabriqué d'un seul tenant lors d'un procédé de coulée par injection, sachant que les bords (3, 6) des deux parois (2, 4) sont reliés à une charnière à film (1).
